# EUROPEAN PATENT SPECIFICATION

(11) **EP 4 056 161 B1**
(45) Date of publication and mention of the grant of the patent: **01.11.2023**
(21) Application number: 21216159.0
(22) Date of filing: 14.09.2017
(51) Int. Cl.: A61H 21/00, A61H 23/00, A61B 5/00, E03D 11/13

(54) **A SYSTEM FOR ADAPTIVE TREATMENT OF DISORDERS IN THE GASTROINTESTINAL TRACT**
EIN SYSTEM ZUR ADAPTIVEN BEHANDLUNG VON ERKRANKUNGEN DES MAGEN-DARM-TRAKTS
SYSTÈME DE TRAITEMENT ADAPTATIF DES TROUBLES DU TRACTUS GASTRO-INTESTINAL

(30) Priority: 21.09.2016 GB 201616044; 21.08.2017 CN 201721046328 U
(43) Date of publication of application: 14.09.2022
(62) Divisional of application: 17852496.3
(73) Proprietor: Vibrant Ltd., 2069206 Yokne'am (IL)
(72) Inventor: BEN-TSUR, Lior, 4267000 Netanya (IL); MOLNAR, Shai, 20162 Shorashim (IL); SHABAT, Roni, 1892500 Kfar Yehezkel (IL); LAMPERT, Shalom, 2152041 Maalot (IL)
(74) Representative: Harrison IP Limited

(56) References cited:
- WO-A1-2013/121276
- CN-A- 105 434 155
- US-A1- 2009 326 514

## Description

### FIELD OF THE INVENTION

The present invention relates in general to medical devices and treatment systems, particularly to systems for adapting a treatment protocol of ingestible capsules introducible to the gastrointestinal (GI) tract of a subject to the subject's response to one or more previous treatment protocols.

### BACKGROUND

CN105434155 discloses an intelligent vibration capsule system with the gastrointestinal motility adjusting function. The system comprises a miniature electronic vibration capsule, a portable ex-vivo controller and an ex-vivo data processing system. The miniature electronic vibration capsule is used for being swallowed, stimulates the gastrointestinal tract through an internal vibrator, receives a magnetic field generated by the ex-vivo controller and transmits the intensity of the magnetic field outside the body through wireless data. The portable ex-vivo controller is arranged outside the body and used for receiving, recording and analyzing the data transmitted by the miniature electronic vibration capsule and displaying the position of the miniature electronic vibration capsule inside the body. The ex-vivo data processing system reads data stored in the portable ex-vivo controller and analyzing and processing the data to acquire the pressure condition and the passing time of the miniature electronic vibration capsule in the gastrointestinal tract and a gastrointestinal tract model. The mechanical vibration capsule can be intelligently activated, the gastrointestinal tract can be stimulated to be induced to shrink and wriggle, and therefore constipation is relieved and treated.

WO2013/121276 discloses gastrointestinal capsule and treatment methods utilizing such a capsule, the capsule including: (a) a capsule housing having a longitudinal axis; (b) at least one thrusting mechanism, disposed within the housing, the thrusting mechanism adapted to exert radial forces on the housing, in a radial direction with respect to the axis, such that, when the capsule is disposed within a gastrointestinal tract of a user, and the mechanism is in an active mode, the gastrointestinal capsule exerts forces against, or in a direction of, the walls of the tract; and (c) a power supply adapted to power the mechanism, wherein a ratio of the radial forces to axial forces exerted in an axial direction with respect to the axis, on the housing, by the thrusting mechanism, is at least 1:1.

### SUMMARY OF THE INVENTION

In accordance with the invention, there is provided a system for treatment of a disorder in a gastrointestinal (GI) tract of a subject as set forth in claim 1 of the appended claims.

Preferred features of the invention are set forth in claims to 13 of the appended claims.

### BRIEF DESCRIPTION OF THE FIGURES

The foregoing discussion will be understood more readily from the following detailed description of the invention, when taken in conjunction with the accompanying Figures (1-12), in which:
Figure 1 is a schematic block diagram of a system for treatment of a disorder in the gastrointestinal tract of a subject;
Figures 2A and 2B together are a schematic flowchart of a method (which is not covered by the invention) for treatment of a disorder in the gastrointestinal tract of a subject, the method (which is not covered by the invention) utilizing the system of Figure 1;
Figures 3A, 3B, and 3C are, respectively, a partially cut away side plan view, a partially cut away perspective view, and a top plan view of a toilet-bowl mounted sensor for capturing and identifying an ingestible capsule, the toilet-bowl mounted sensor forming part of the system of Figure 1;
Figure 4 is a sectional view of another embodiment of a receptacle, forming part of a toilet-bowl mounted sensor, in a stand-by mode;
Figures 5A and 5B are, respectively, a perspective view and a sectional view of the receptacle of Figure 4 in an operational mode;
Figure 6 is a partial sectional view of the receptacle of Figure 4 in a capsule capturing mode;
Figure 7 is a sectional view of the receptacle of Figure 4 in a capsule releasing mode; and
Figure 8 is a schematic flowchart of a method (which is not covered by the invention) for capturing and identifying an ingestible capsule that has been expelled into a toilet bowl, the method (which is not covered by the invention) utilizing the toilet-bowl mounted sensor of any one of Figures 3A-7.

### DESCRIPTION OF THE PREFERRED EMBODIMENTS

The principles of the system and method (which is not covered by the invention) for treatment of a disorder in the gastrointestinal tract of a subject, and the toilet-bowl mounted sensor for capturing and identifying an ingestible capsule that has been expelled into a toilet-bowl and method of use (which is not covered by the invention) thereof, may be better understood with reference to the drawings and the accompanying description.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

For the purposes of this application, the term "subject" relates to a human subj ect.

For the purposes of this application, the term "vibrating ingestible capsule" relates to an ingestible capsule adapted to at least intermittently vibrate, for a cumulative duration of at least one minute, in accordance with a vibration protocol of the capsule, such that, when the capsule is disposed in the gastrointestinal (GI) tract of a subject and is in operative mode, the vibrations are delivered to a wall of the GI tract of the subject, so as to provide mechanical stimulation to the GI tract of the subject. Typically, such mechanical stimulation is provided so as to treat GI disorders such as constipation.

For the purposes of this application, the term "programmable vibrating ingestible capsule" relates to a vibrating ingestible capsule adapted to vibrate in accordance with vibration protocol, the vibration protocol adapted to be programmed into the capsule by a capsule control unit. Specifically, the vibration protocol of a programmable vibrating ingestible capsule may be changed by re-programming the capsule to implement a different vibration protocol.

For the purposes of this application, the term "programmed vibrating ingestible capsule" relates to a vibrating ingestible capsule adapted to vibrate in accordance with a vibration protocol, the vibration protocol being programmed into the capsule, for example by the manufacturer thereof. The programmed vibration protocol is fixed, and the capsule cannot be re-programmed.

For the purposes of this application, the term "capsule control unit" relates to a device adapted to produce a vibrating ingestible capsule and/or to activate a vibrating ingestible capsule. Typically, the capsule control unit is adapted to receive a vibration protocol, and to transmit the vibration protocol to a vibrating ingestible capsule, thereby to program the capsule to implement the vibration protocol. For example, the capsule control unit may provide the vibration protocol to a processor of the programmable vibrating ingestible capsule by means of remote communication, such as by short range wireless communication, and may provide the protocol as a list of parameters, as executable code, or in any other manner suitable for the capsule to implement the vibration protocol. For activation, the capsule control unit typically is adapted to transmit an activation signal to a vibrating ingestible capsule, indicating to a processor of the capsule that the vibration protocol should be initiated. In some cases, the capsule control unit is also adapted to receive a signal from the capsule, indicating that the programmed vibration protocol and/or the activation instruction have been received.

For the purposes of this application, the term "short range wireless communication method" relates to any wireless communication method or protocol in which signals are communicated up to a maximum range of at most 1 kilometer, at most 500 meters, at most 300 meters, at most 200 meters, at most 100 meters, at most 50 meters, or at most 10 meters such as, for example, Bluetooth communication, Wi-Fi communication, RFID signal communication, low frequency magnetic field, and the like.

For the purposes of this application, the term "intermittently activated vibration engine" refers to a vibration engine which is adapted to vibrate at certain times and not to vibrate at other times, the activation times being selected by a processor or other control unit controlling the vibration engine, such as a processor of a vibrating ingestible capsule of which the vibration engine forms part.

For the purposes of this application, the term "vibration protocol" relates to a protocol specifying vibration parameters of an intermittently activated vibration engine of a vibrating ingestible capsule. Typically, the vibration protocol relates to an activation delay for initiating vibration (a duration between activation of the capsule and the first activation of the vibration engine) which is typically in the range of 0-24 hours, a vibration rate (number of vibration cycles per hour) which is typically in the range of 1-300 cycles per hour, a vibration period and a repose period for each vibration cycle, a vibration frequency which is typically in the range of 1-500Hz, an amount of force to be exerted by the vibrations, a set of vibration and non-vibration durations (i.e. a set specifying that the capsule vibrates at the vibration rate for a certain duration or a certain number of cycles, then rests for a certain duration, vibrates again for a certain duration or number of cycles, and so forth), and the like. The vibration and non-vibration durations may be defined as absolute times, or may be relative to ingestion or activation of the capsule. For example, a treatment protocol may indicate that the activation delay is 4 hours (i.e. intermittently activated vibration engine should begin vibration 4 hours after activation or ingestion of the capsule), and that following the activation delay the capsule should vibrate at a frequency of 150Hz and at a duty cycle of 60%, for a duration of 2 hours, stop vibrating for 2 hours, and subsequently resume vibration for a duration of four hours, at a frequency of 250Hz and at a duty cycle of 85%, each cycle having a vibration period of 3 seconds and a repose period of 26 seconds. As another example, the activation delay is 8 hours, and subsequently vibration begins at 200Hz at a 40% duty cycle in repetitive cycles having a 1 second vibration period followed by a 27 second repose period. After each five cycles of vibration, the vibration frequency is increased by 2Hz, and the duty cycle is increased by 1%.

For the purposes of this application, the term "treatment procedure" relates to parameters of a treatment using vibrating ingestible capsules, which are typically defined by a treating physician or medical practitioner. For example, the treatment procedure may include the number of capsule to be taken in a specific time duration (e.g. 3 capsules per week, 2 capsules per day, etc.), the frequency at which capsules should be taken, the time of day at which capsules should be taken, whether the capsule should be taken with or without food, and the like.

For the purpose of this application, the term "treatment protocol" relates to all aspects of treatment of a subject with a vibrating ingestible capsule, and includes the treatment procedure as well as the vibration protocol to be used for treating the subject.

For the purposes of this application, the term "default treatment protocol" relates to a standard treatment protocol which is typically used when initially treating a subj ect with a vibrating ingestible capsule, or when insufficient information is available for tailoring a specific treatment protocol to the subject.

For the purposes of this application, the terms "default vibration protocol" relates to a standard vibration protocol which is typically used when initially treating a subject with a vibrating ingestible capsule, or when insufficient information is available for tailoring a specific treatment protocol to the subject. In some cases, the default vibration protocol is the vibration protocol used by programmed vibrating ingestible capsules.

For the purposes of this application, the term "electronically obtained", when relating to a treatment protocol, relates to a treatment protocol which was generated by or determined by a machine based on electronic information, such as information included in a computerized or electronic profile of a subject and/or data included in a database, without direct human input or intervention.

For the purposes of this application, the term "successful treatment", and variations thereof, relates to treatment of a subject with one or more capsules implementing a specific treatment protocol such that during or following the treatment, the symptoms of the subject are improved by a significant degree, as defined by medical practices. For treatment of chronic constipation, successful treatment is treatment that results in an increase of at least one bowel movement per two-weeks, an increase of at least one bowel movement per week, an increase of three bowel movements per two-weeks, or an increase of at least two bowel movements per week, on average.

For the purposes of this application, the term "decision maker" relates to any entity capable of applying logic to a proposed treatment protocol, in order to determine whether or not the treatment protocol is suitable for treatment of a subject, and/or whether the treatment protocol must be modified in order to be suitable for treatment of the subject. The decision maker may be a human, or a machine including suitable artificial intelligence or logic components.

For the purposes of this application, the term "electronic decision maker" relates to a decision maker which is fully based on artificial intelligence or logic, and which makes decisions regarding the suitability of a proposed treatment protocol for treatment of a subject without direct input from a human, only based on artificial logic and/or intelligence. The electronic decision maker is typically limited to decisions within a scope determined by a human, such as a medical practitioner, prior to use of the electronic decision maker. For example, a doctor may define a range of vibration protocols which is acceptable for use for a subject, and the electronic decision maker may make decisions within that range, but would refer to a human decision maker if decisions are required that are outside of the predefined range.

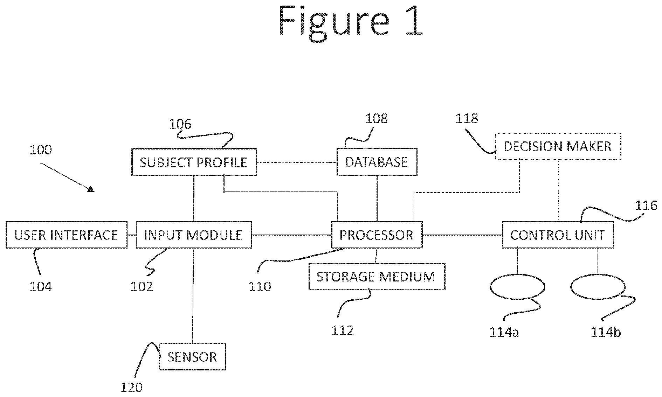

Referring now to the drawings, Figure 1 is a schematic block diagram of a system 100 for treatment of a disorder in the gastrointestinal tract of a subject.

As seen in Figure 1, system 100 includes at least one input module 102 operative to receive input from at least one of the subject, a medical practitioner or medical personnel treating the subject, and a care giver of the subject, such as a parent, guardian, or personal medical aide, for example via a user interface **104.** The input module **102** may further be operative to receive input from one or more sensors, as described in further detail hereinbelow. In some embodiments, separate input modules **102** and/or different user interfaces **104** are used for different input providers.

The input module **102** may be any suitable input module, and may include, for example, a receiver or transceiver configured to receive the input as a communication signal from a remote location, such as from the one or more sensors, or a keyboard or touchpad configured to receive input entered directly thereinto, for example by the subject, a caretaker of the subject, medical personnel treating the subject, or any other input provider.

A computer readable memory stores a subject profile **106** for the subject, including subject data. The subject profile **106** may be functionally associated with the input module **102,** and the subject data may be provided to the subject profile **106** as input, via one of more input module **102.** For example, the subject, the medical practitioner or medical personnel treating the subject, or the care giver of the subject may provide the subject data, for example as an initial input.

In some embodiments, the subject data includes demographic information of the subject, such as the subject's name, identification number such as a social security number or passport number, address, date of birth, age, gender, contact information, and emergency contact information. In some embodiments, the subject data includes medical information of the subject, such as the subject's medical history and particularly the medical history of constipation and or gastrointestinal diseases, medications and/or treatments previously tried by the subject, particularly for constipation and gastrointestinal diseases, results of medical examination such as etiology of constipation, results from physical examination including digital rectal examinations, results from gastrointestinal explorations such as transit or motility studies, anorectal manometry, balloon test expulsion, or other gastrointestinal explorations, information relating to allergies, chronic diseases, medications currently being used, and the like. In some embodiments, the subject data may also include information relating to the subject's lifestyle, such as diet, water intake, and physical activity. In some embodiments, for female subjects, the subject data may also include obstetric medical history.

In some embodiments, the subject data may include guidelines for treatment of the subject, or a "treatment safe zone" or "self adapting zone" for the subject, such as, for example, a range of vibration protocols suitable for treatment of safely treating the subject, which may be used by an electronic decision maker as described in further detail hereinbelow.

In some embodiments, the system includes a database **108,** which may be functionally associated with one or more input module **102** and/or with subject profile **106,** includes data relating to subjects treated for disorders in the gastrointestinal tract. In some embodiments, data is input into the database **108** directly by an input provider, such as one or more subjects, a medical practitioner, medical personnel, a researcher or research facility, and the like, for example via input module **102** and user interface **104.** In some embodiments, the data is input into the database **108** automatically, for example directly from the subject profile **106,** as described in further detail hereinbelow.

In some embodiments, data included in database **108** relates to at least one other subject, and preferably to a plurality of subjects other than the subject. In some embodiments, the data included in database **108** includes, for each other subject, demographic information such as gender, age, date of birth, and the like, as well as information relating to disorders of the gastrointestinal tract experienced by the other subject, to treatments provided for such disorders, and to the other subject's response to such treatments. In some embodiments, the data included in database **108** may be substantially parallel to the subject data. Specifically, in some embodiments, the data included in the database lists, for each of the other subjects, one or more vibration protocols, treatment procedures, and/or treatment protocols used to treat the other subject, and the user's response to treatment with such protocols and procedures.

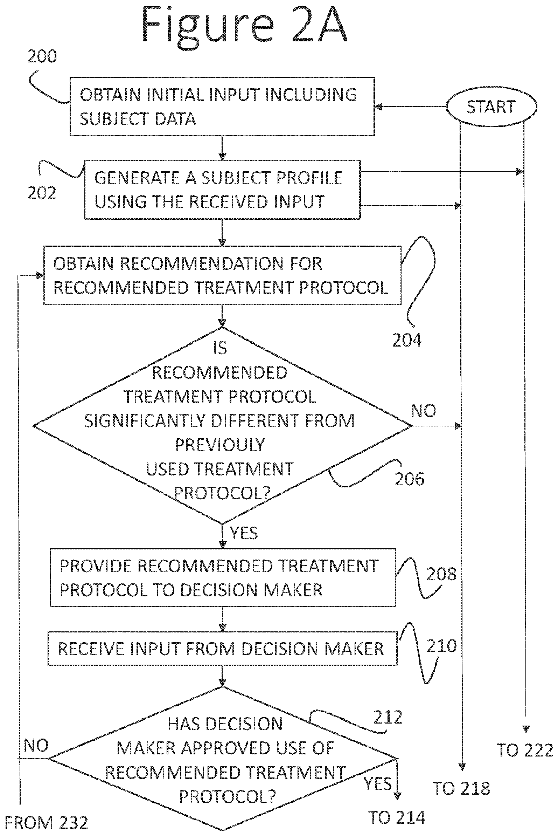

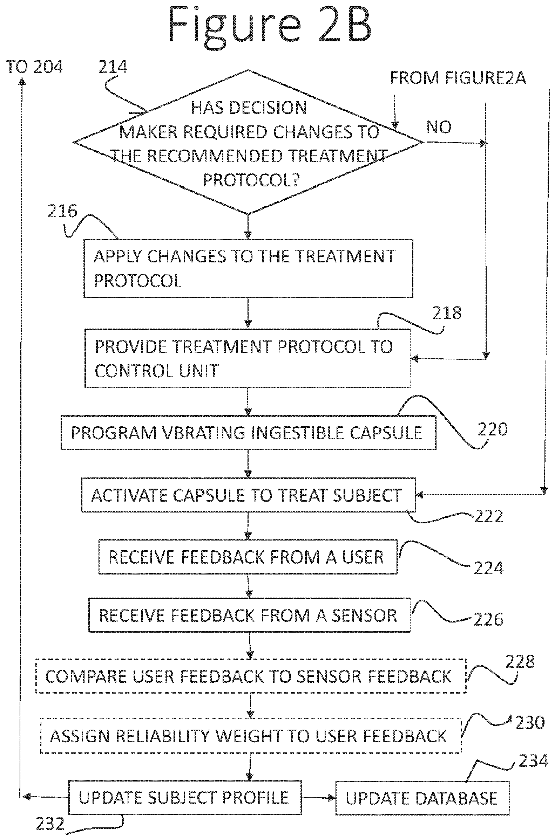

A processor **110** is functionally associated with subject profile **106** and with database **108,** and may further be functionally associated with input module **102.** Processor **110** is functionally associated with a computer readable storage medium **112** storing instructions, which may be carried out by the processor **110,** as explained in further detail hereinbelow with respect to Figure 2.

In some embodiments, processor **110** is configured to receive, for example via input module **102** or via subject profile **106,** feedback relating to a response of the subject to treatment with a specific treatment protocol, a specific vibration protocol, or specific treatment procedures. For example, the feedback may indicate times at which the subject experienced bowel movements following the treatment, a physical feeling experienced by the subject during or immediately after the treatment, or one or more characteristics of fecal matter excreted by the subject during or following the treatment.

In some embodiments, the feedback is received from a human, such as from the subject, a medical practitioner or medical treating the subject, or a care-giver of the subject. In some embodiments, the feedback is received from a sensor, as described in further detail hereinbelow.

In some embodiments, the processor **110** is further configured to assign a weight, or a reliability score, to such feedback, as described in further detail hereinbelow.

In some embodiments, processor **110** is configured to electronically obtain an initial recommendation for an initial recommended treatment protocol for the subject. In some embodiments, the initial recommendation may be a default initial recommendation. In some embodiments, the initial recommendation may be based on at least one selected characteristic included in the subject data. In some embodiments, the initial recommendation may be based on data included in the database **108,** for example data relating to other subjects sharing the selected characteristic(s) with the subj ect.

In some embodiments, processor **110** is configured to electronically obtain an updated recommendation for an updated recommended treatment protocol for the subject, based on received feedback relating to a response of the subject to a previously used treatment protocol, on at least one characteristic included in the subject data and/or on data included in the database **108,** for example data relating to other subjects sharing the characteristic with the subject or sharing the experienced response with the subject.

The updated recommendation may change one or more parameters of the vibration protocol or of the treatment protocol, relative to a previous recommendation. For example, the updated recommendation may change the vibration frequency, the delay time until starting the vibration protocol, the duty cycle, the segment in the GI tract in which the capsule is intended to vibrate, the length of the vibration time in each vibration cycle, the rest time of each vibration cycle, and/or the vibration intensity.

The system **100** further includes a first vibrating ingestible capsule **114a,** adapted to implement a first vibration protocol for delivering vibrations, or mechanical stimulation, to a wall of the GI tract of the subject thereby to treat the gastrointestinal disorder of the subject, the first vibration protocol forming part of a first treatment protocol for the subject. The system further includes at least one second programmable vibrating ingestible capsule **114b,** adapted to be programmed to implement a second vibration protocol delivering vibrations, or mechanical stimulation, to a wall of the GI tract of the subject thereby to treat the gastrointestinal disorder of the subject, the second vibration protocol forming part of a second treatment protocol for the subject. As explained in further detail hereinbelow, in some embodiments, the first and/or second vibration protocols and/or treatment protocols may be identical to, or may be based on, recommended vibration protocols and/or treatment protocols electronically obtained by processor **110.**

Each of capsules **114a** and **114b** may include a sensor module including one or more sensors; a timer; an intermittently activated vibration engine; a processor, functionally associated with the sensor(s) in the sensor module, with the timer, and with the vibration engine; a receiver or transceiver functionally associated with the processor; and at least one power source providing power to the sensor module, the timer, the vibration engine, the processor, and/or the receiver/transceiver.

In some embodiments, the sensors included in the sensor module may sense conditions in the vicinity of the capsule. As such, the sensors may include: a pH sensor adapted to sense the pH in the vicinity of the capsule; a light sensor adapted to sense a degree of illumination in the vicinity of the capsule; a pressure sensor adapted to sense pressure applied to the capsule; and/or an orientation sensor, such as an accelerometer, adapted to sense the three dimensional orientation of the capsule.

The power source of capsules **114a** and **114b** may be any suitable power source, such as, for example, one or more alkaline or silver oxide batteries, lithium batteries, primary batteries, rechargeable batteries, capacitors and/or super capacitors.

In some embodiments, the capsules **114a** and **114b** may be substantially as described in U.S. Patent Application Publication No. 2015/0073315 and in U.S. Patent No. 9,078,799.

In some embodiments, first vibrating ingestible capsule **114a** may be a programmed vibrating ingestible capsule, programmed, for example at a time of manufacturing thereof, to implement a specific vibration protocol as the first vibration protocol. In other embodiments, the first vibrating ingestible capsule **114a** is a first programmable vibrating ingestible capsule **114a,** adapted to be programmed to implement the first vibration protocol.

In some embodiments, the first treatment protocol may be a default treatment protocol, used as the standard initial treatment protocol for subjects suffering from any disorder, or from a specific disorder, of the gastrointestinal tract, and may define a default vibration protocol as the first vibration protocol.

A capsule control unit **116,** functionally associated with processor **110** and with at least one programmable vibrating ingestible capsule, is adapted to receive a vibration protocol and to program a programmable vibrating ingestible capsule to implement the received vibration protocol. In some embodiments, the capsule control unit is adapted to receive a treatment protocol including treatment procedures and a vibration protocol and to program the programmable vibrating ingestible capsule to implement the received vibration protocol.

In some embodiments, capsule control unit **116** may include a dedicated processor; a programming module; a verification and activation module; and/or a transceiver or other communication module. The capsule control unit may further include at least one power source providing power to components of the capsule control unit.

In some embodiments, the capsule control unit **116** further includes a user interface, adapted to provide output to the subject or to another user associated with the subject, such as a medical practitioner or care giver. In some such embodiments, the capsule control unit **116** may prompt the subject, via the user interface, to ingest one or more capsules in accordance with the treatment protocol received by the capsule control unit.

Capsule control unit **116** may be any suitable control unit, including a suitably programmed computing device such as a smartphone, a tablet computer, laptop computer, desktop computer, and the like, or may be a dedicated control unit, e.g. forming part of another medical device.

In system **100,** capsule control unit **116,** or a dedicated processor thereof, is adapted to receive from processor **110** the second treatment protocol, for example via a transceiver of the capsule control unit **116,** and to program the second programmable vibrating ingestible capsule **114b** to implement the second vibration protocol defined in the second treatment protocol. In some embodiments, in which first vibrating ingestible capsule **114a** is programmable, capsule control unit **116** is further adapted to similarly receive the first treatment protocol, from processor **110** or from another source, and to program the first vibrating ingestible capsule **114a** to implement the first vibration protocol defined in the first treatment protocol.

In some embodiments, the programming module of capsule control unit **116** is configured to receive the second vibration protocol from the processor of the capsule control unit, and to program a processor of second programmable vibrating ingestible capsule **114b** to implement the second vibration protocol upon activation thereof or upon ingestion of the second vibrating ingestible capsule. Similarly, in embodiments in which first vibrating ingestible capsule **114a** is programmable, the programming module of capsule control unit **116** is configured to receive the first vibration protocol from the processor of the capsule control unit, and to program a processor of first vibrating ingestible capsule **114a** to implement the first vibration protocol upon activation thereof or upon ingestion of the first vibrating ingestible capsule.

In some embodiments, the programming module of capsule control unit **116** may include a transmitter, or may be functionally associated with a transmitter, for transmission of the first and/or second vibration protocol to a receiver/transceiver of capsules **114a** and/or **114b,** for example using a short range wireless communication method.

In some embodiments, the programming module of capsule control unit **116** transmits the vibration protocol to the capsule **114a** or **114b** as a sequence of parameter signals, such as digits, indicating time frames from activation or from ingestion and vibration parameters to be used at those time frames. In some embodiments, the programming module of capsule control unit **116** transmits the vibration protocol to the capsule **114a** or **114b** as executable code to be used by the processor of the capsule to automatically activate the vibration engine thereof in accordance with the vibration protocol.

In some embodiments, a memory element within the capsule **114a** or **114b** includes multiple vibration protocols which may be effected by the capsule, and the programming module of capsule control unit **116** transmits to the capsule and indication of a specific one of the multiple vibration protocols to be used, such as a vibration protocol identifier.

In some embodiments, the activation module of the capsule control unit **116** is configured to activate the first and/or second programmable vibrating ingestible capsule for operation.

In some cases, the activation includes activating the capsule to identify ingestion thereof by the subject, and subsequently to being implementation of the vibration protocol programmed therein. In other embodiments, activation of the capsule includes immediate activation of the timer to start counting time till activation of the vibration engine. Such embodiments assume that the capsule is activated shortly before the subject ingests the capsule.

In some embodiments, the activation module of capsule control module **116** includes a transmitter, or is functionally associated with a transmitter or transceiver, and activates the capsule by providing an activation signal thereto.

In some embodiments, the activation module also includes a verification module, configured to verify that the capsule received the vibration protocol transmitted thereto and is capable of effecting the vibration protocol without error. In some embodiments, the verification module includes, or is functionally associated with a receiver, receiving from the capsule a verification signal indicating that the capsule has received the vibration protocol. In some embodiments, the verification module includes a vibration sensor, sensing the capsule vibrating in accordance with a specific vibration pattern indicative of verification thereof.

In some embodiments, the processor **110** and the capsule control unit **116** are functionally associated with a decision maker **118,** which is adapted to receive, for example from processor **110,** a suggestion or recommendation for a treatment protocol or a vibration protocol, and to approve, reject, or modify the suggested or recommended protocol for treatment of the subject.

In some embodiments, the decision maker may be a human decision maker, such as a medical practitioner or treating gastroenterologist, who may receive the suggested or recommended treatment protocol or vibration protocol by the protocol being presented on a display visible to the human decision maker, or by receiving an electronic message, such as an email message, specifying the recommended or suggested treatment protocol or vibration protocol.

The human decision maker may approve, reject, or modify the received treatment protocol or vibration protocol by interacting with a user interface **104** of one of input modules **102,** or by interacting directly with capsule control unit **116,** for example with an input module or user interface thereof.

In other embodiments, the decision maker **118** is an electronic decision maker, such as a suitably equipped computer or server, which is adapted to electronically receive the suggested or recommended treatment protocol or vibration protocol, and to approve, reject, or modify the suggested or recommended treatment protocol or vibration protocol. In some embodiments, the logic applied by the electronic decision maker may be learned over time, for example based on the results that similar treatment had for other subjects. In some embodiments, the electronic decision maker may include an artificial intelligence component.

In some embodiments, decisions made by an electronic decision maker are limited to be within certain limitations, or within a "self adapting zone" predefined by a human decision maker, such as safety regulations defined by a manufacturer of the capsule and/or medical limitations defined by a medical practitioner. For example, the manufacturer may indicate that the frequency of vibrations has an upper limit of 1000Hz, in which case the decision maker will not permit, or approve, any protocol requiring vibration at a frequency greater than 1000Hz. As another example, a medical practitioner may indicate that no changes can be made to treatment procedures, in which case the electronic decision maker would make decisions relating to the vibration protocol, but would defer any decisions relating to the treatment procedures to a human decision maker, for example via an electronic message.

In some embodiments, the limitations are based on the structure and/or functionality of the capsule, such as safety limitations defined by the manufacturer. In such embodiments, the same limitations apply to multiple subjects, and multiple capsules. In some embodiments, the limitations are defined by the medical practitioner based on the medical state of the subject, and are specific to the subject being treated. Such limitations may be provided as input by the medical practitioner, for example as part of the subject data stored in the subject profile.

In some embodiments, the electronic decision maker may be functionally associated with the capsule control unit **116,** and may transmit the approved or modified treatment protocol or vibration protocol directly to the capsule control unit **116.** In other embodiments, the electronic decision maker provides the approved or modified treatment protocol or vibration protocol to processor **110,** which may transmit the treatment protocol to capsule control unit **116** as described hereinabove.

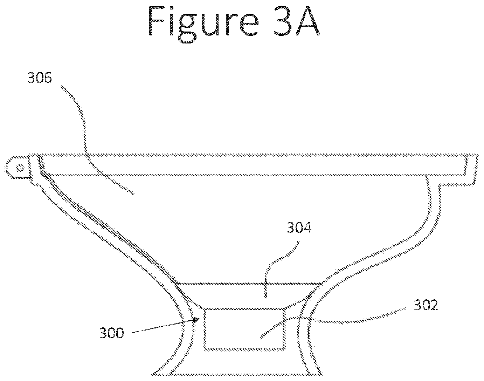

In some embodiments, processor **110** and/or subject profile **106** is further associated with at least one sensor **120,** which is adapted to sense, and provide to processor **110** or to subject profile **106,** information regarding a vibrating ingestible capsule being expelled from the body of the subject. In some embodiments, the sensor comprises a toilet-bowl mounted sensor. The toilet bowl mounted sensor may be any suitable sensor, such as any one of the sensors described hereinbelow with reference to Figures 3A to 8, or a toilet-bowl mounted sensor known in the art, for example as described in US Patent Application Publication No. 2009/0326514.

The information provided by sensor **120** may include a time at which the vibrating ingestible capsule was expelled from the body of the subject, and/or an identification of the expelled capsule.

Reference is now additionally made to Figures 2A and 2B, which, together, are a schematic flowchart of a method for treatment (which is not covered by the invention) of a disorder in the gastrointestinal tract of a subject, the method (which is not covered by the invention) utilizing the system of Figure 1.

As seen, and as described in further detail hereinbelow, the method (which is not covered by the invention) may start at step **200,** at step **218,** or at step **222.**

In some embodiments, the method (which is not covered by the invention) begins at step **200,** by obtaining initial input including subject data for a subject to be treated using the method (which is not covered by the invention). The subject data typically includes demographic information and/or medical history information for the subject, required for initiating subject profile **106.** In some embodiments, the input may additionally include information relating to other subjects, such as information required for creating or updating database **108.** In some embodiments, the input may further include limitations defining a "self adapting zone" for an electronic decision maker, as described hereinabove.

As mentioned hereinabove with reference to Figure 1, the input may be received from the subject, from a medical facility or medical practitioner treating the subject, or from a caretaker of the subject. In some embodiments, the input is provided to input module **102** via a user interface **104** which is local to system **100,** while in other embodiments the input may be provided via a remote user interface **104,** and communicated to input module **102** or to processor **110** using a suitable communication method.

At step **202,** a subject profile, such as subject profile **106** described hereinabove with respect to Figure 1, is generated for the subject, for example by processor **110,** based on the input relating to the subject.

Turning to step **204,** it is seen that a recommendation for a recommended treatment protocol for the subject is obtained. In some embodiments, the recommendation is electronically obtained by processor **110.** In some embodiments, the recommendation is based on information included in the subject profile **106.**

For example, in an initial treatment round, the subject profile may indicate that the motility in the subject's digestive system is slower than the average digestive system, such that the food reaches the colon after a longer time than the average 10 hours. In such embodiments, processor **110** may recommend a treatment protocol having a long enough delay time so as to ensure that the capsule vibrates when it has reached the subject's large intestine, and does not vibrate prematurely.

In some embodiments the recommendation may also be based on data obtained from database **108,** the data relating to one or more other subjects. For example, in an initial treatment round, the subject profile **106** may indicate that the subject is a female aged 55. Processor **110** may access database **108** to identify treatment protocols and/or vibration protocols that were successfully used to treat other females having similar ages, for example aged 50-60. One such identified protocol may be suggested as the recommended treatment protocol for the subject, or a new treatment protocol, including characteristics of a number of such identified protocols, may be generated as the recommended treatment protocol for the subject.

As described in further detail hereinbelow, the method (which is not covered by the invention) described herein is iterative, and subject profile **106** may be updated to reflect the response of the subject to treatment with a specific treatment protocol. As such, in subsequent treatment rounds, processor **110** may use data relating to such responses when generating the recommendation for the next treatment protocol to be used.

Returning to the example provided above, the subject profile **106** may, in a later treatment round, indicate that the subj ect's condition did not improve when treated with a vibration protocol providing capsule vibration at a frequency of 150Hz. The processor may then recommend an updated vibration protocol having a different vibration frequency.

Selection of a subsequent treatment protocol may also be based on data from the database, relating to at least one other user. In the example above in which the subject's condition did not improve when treated with a vibration protocol providing capsule vibration at a frequency of 150Hz, the processor may, when identifying in database **108** potential treatment protocols, limit the identified protocols to ones providing vibration at a higher frequency, provided that the frequency is within a predetermined permissible range, such as up to 250Hz. As another alternative, the processor may identify treatment protocols that were successfully used to treat females aged 50-60 which did not respond well to treatment with capsule vibration at a frequency of 150Hz.

When updating the vibration protocol or treatment protocol in a second or subsequent treatment round, the updated recommended treatment protocol may change one or more parameters of the vibration protocol or of the treatment protocol, relative to a previous recommendation. For example, the updated recommendation may change the vibration frequency, the delay time until starting the vibration protocol, the duty cycle, the segment in the GI tract in which the capsule is intended to vibrate, the length of the vibration time in each vibration cycle, the rest time of each vibration cycle, and/or vibration intensity.

As seen at step **206,** the processor **110** may then check whether or not the obtained recommended treatment protocol is significantly different from a previously used treatment protocol. Naturally, in an initial iteration of the method (which is not covered by the invention) described herein, step **206** would be obviated. In some embodiments, a treatment protocol would be considered 'significantly different' from another treatment protocol if the vibration protocol and/or the treatment procedures defined by the treatment protocol, or any portion thereof, is not in the range of allowed treatment protocols and/or characteristics defined for the subject, as described hereinabove.

If the recommended treatment protocol is significantly different from the treatment protocol used in a previous round of treatment, at step **208** the recommended treatment protocol is provided by processor **110** to decision maker **118,** which may be a human decision maker, such as a medical practitioner treating the subject, or may be an electronic decision maker, as described hereinabove.

As seen at step **210,** input from the decision maker is received by processor **110.** The decision maker may require that the recommended treatment protocol be modified prior to treating the subject, or may indicate that the recommended treatment protocol, or a similar treatment protocol based on the recommended treatment protocol, should not be used for the subject, and such requirements may be provided as the input. In embodiments in which the decision maker is human, the input may be provided via user interface **104** and input module **102.**

At step **212,** processor **110** checks whether or not the decision maker has approved treating the subject using a treatment protocol which is based on the recommended treatment protocol. If processor **110** identifies that the decision maker has not approved use of a treatment protocol based on the recommended protocol, the method returns to step **204** for selection of another recommended treatment protocol.

Otherwise, if processor **110** identifies that the decision maker approved use of the recommended treatment protocol, at step **214** the processor checks whether or not the decision maker has required making any changes to the recommended treatment protocol in order to obtain the treatment protocol to be used. As seen at step **216,** if such changes are required, processor **110** applies the changes to the recommended treatment protocol, thereby to generate the treatment protocol to be used.

Once the treatment protocol is generated at step **216,** the generated treatment protocol, or, in some embodiments, only the vibration protocol defined thereby, is provided by processor **110** to capsule control unit **116** at step **218.** Alternately, if no changes were required to the recommended treatment protocol (at step **214**), or if the recommended treatment protocol is not significantly different from the treatment protocol used in an immediately previous round of treatment (at step **208**), the treatment protocol to be used is set to be identical to the recommended treatment protocol, and the treatment protocol, or only the vibration protocol defined thereby, is provided by processor **110** to capsule control unit **116,** at step **218.**

As another alternative, the method (which is not covered by the invention) may start from step **218,** for example by providing to the capsule control unit **116** a default treatment protocol or a default vibration protocol to be used in a first treatment iteration. In such embodiments, the default treatment protocol or the default vibration protocol may be provided by the manufacturer of the capsule or by a medical practitioner for any subject beginning treatment using the system of Figure 1, regardless of the subject's profile or of any data collected in the database regarding other subjects. In other embodiments, the method may begin with steps **200** and **202** so as to create the subject profile, and may then proceed directly to step **218** wherein the default treatment protocol or default vibration protocol is provided to the capsule control unit **116.**

Once the capsule control unit **116** has received the vibration protocol to be used, possibly as part of the treatment protocol to be used, at step **220** the capsule control unit **116** programs a programmable vibrating ingestible capsule, such as capsule **114a** or **114b,** to implement the vibration protocol upon activation of the capsule or upon ingestion thereof, substantially as described hereinabove with respect to Figure 1. In some embodiments, the capsule control unit **116** also provides output to the subject or to a caretaker thereof, for example via a display or other user interface, the output indicating the treatment procedures defined in the treatment protocol to be used.

As seen at step **222,** once the programmable vibrating ingestible capsule (e.g. **114a** or **114b**) has been programmed to implement the vibration protocol, the capsule is activated, for example by the activation module of capsule control unit **116** as described hereinabove, and is provided to the subject for ingestion thereof, thereby to treat the subject. As explained hereinabove, the subject or a caretaker thereof is notified of the treatment procedures, and is instructed to ingest the programmed capsule in accordance with the treatment procedures defined in the treatment protocol being used.

In some embodiments, the first vibrating ingestible capsule used to effect the first iteration of treatment is not a programmable capsule, but rather a programmed capsule, programmed to implement a specific vibration protocol, such as the default vibration protocol, for example as described hereinabove with reference to capsule **114a** of Figure 1. In some such embodiments, the method may begin at step **222,** by activation of the programmed capsule and treatment of the subject therewith. In other embodiments, the method (which is not covered by the invention) may begin with steps **200** and **202** so as to create the subject profile, and may then proceed directly to step **222** wherein the programmed capsule is activated and used to treat the subject.

During treatment of the subject, or following such treatment, processor **110** receives feedback from a user, which may be the subject, a medical practitioner treating the subject, and/or a caretaker of the subject, regarding the subject's response to the treatment, as seen at step **224.** In some embodiments, the feedback is provided to processor user interface **104** and input module **102.**

In some embodiments, the feedback may indicate times at which the subject experienced bowel movements during or following treatment in accordance with a specific treatment protocol, or a number of spontaneous bowel movements (SBM) experienced during or following the treatment.

In some embodiments, the feedback may indicate a physical feeling experienced by the subject during or following treatment in accordance with a specific treatment protocol, such as a feeling of complete evacuation following a bowel movement, straining, bloating, gas, flatulence, pain during bowel movements, pain at times other than during a bowel movement, or other adverse events occurring during or following the treatment. For example, the subject may provide feedback indicating that vibration was felt when the capsule was passing through the rectal area of the subject, or that stomach pains were relieved following the treatment.

In some embodiments, the feedback may indicate treatment procedures used by the subject during implementation of the treatment protocol, such as, for example, a time of day at which a vibrating ingestible capsule was ingested.

In some embodiments, the feedback may indicate at least one characteristic of fecal matter excreted by the subject during or following the treatment in accordance with the treatment protocol. For example, the subject may provide feedback indicating the Bristol stool measure of a bowel movement following the treatment.

At step **226,** the processor **110** may receive feedback from one or more sensors **120.** In some embodiments, the sensor is mounted in the toilet-bowl, and provides information regarding expelling of the vibrating ingestible capsule from the body of the subject. In some embodiments, the information includes an indication of a time at which the vibrating ingestible capsule was expelled from the body of the subject. In some embodiments, the information includes an identification of the expelled capsule. In some embodiments, the information includes information regarding fecal matter expelled by the subject, such as for example a Bristol stool measure of the fecal matter, as described hereinbelow.

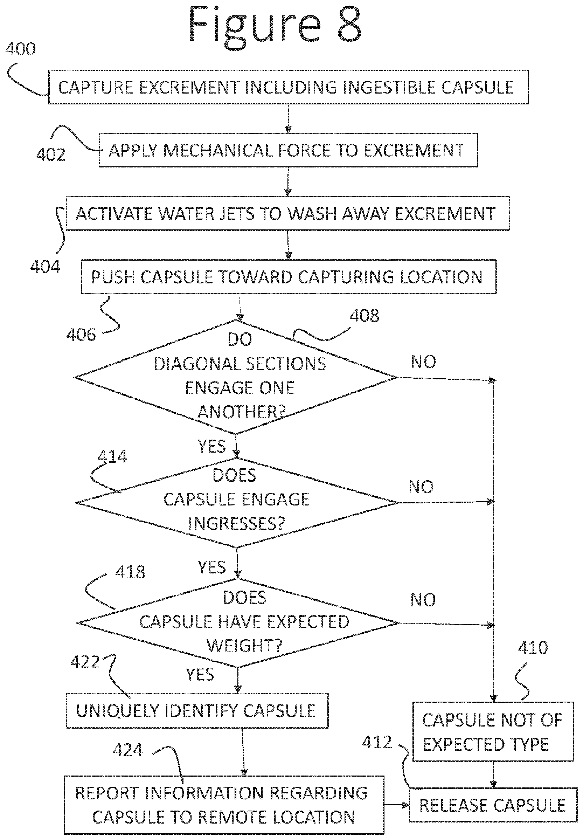

As discussed hereinabove with respect to Figure 1, the toilet-bowl mounted sensor **120** may be any suitable sensor known in the art, such as that described in US Patent Application Publication No. 2009/0326514, or may be a sensor as described hereinbelow with reference to Figures 3A to 7. An exemplary method by which the sensors of Figures 3A to 7 obtain and provide feedback to processor **110** is described hereinbelow with respect to Figure 8.

In some embodiments, processor **110** may optionally compare, or cross-reference, the feedback provided by the subject to the feedback received from the sensor, as seen at step **228,** and may assign a reliability weight to the feedback from the subject based on the results of such comparison, as seen at step **230.**

For example, the processor **110** may compare the times at which the subject reported to have had bowel movements, and the time at which the sensor reported that the capsule was expelled. If the subject did not report a bowel movement at the time that the capsule was expelled, this is indicative of the subject not providing accurate feedback, and as such the sensor based feedback would be assigned a higher reliability weight than the feedback received from the subject.

At step **232,** the processor **110** updates the subject profile **106** based on the feedback provided by the subject, the medical practitioner, and/or the caretaker at step **224,** and/or based on the feedback provided by the sensor **120** at step **226.** In embodiments in which the subject and/or sensor feedback is assigned a reliability score at step **230,** the reliability score is taken into consideration when updating the subject profile, such that a greater significance is given to the more reliable feedback.

Subsequently, the method (which is not covered by the invention) returns to step **204,** and an updated treatment protocol recommendation for the subject is obtained based on the updated user profile and based on data from database **108,** which data relates to at least one other subject.

In some embodiments, following updating of the subject profile **106** at step **232,** the processor **110** creates or updates an entry in database **108** relating to the subject, for example based on the update subject profile **106,** as seen at step **234.**

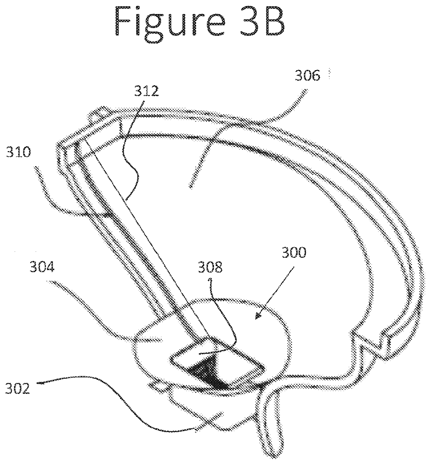

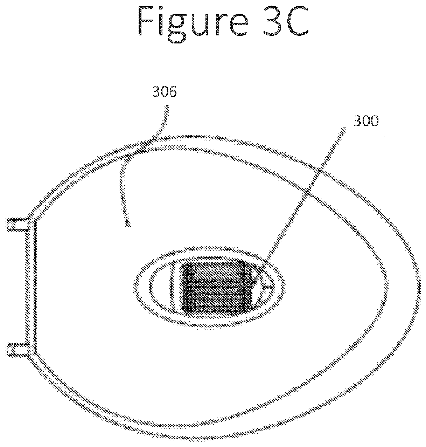

Reference is now made to Figures 3A, 3B, and 3C which are, respectively, a partially cut away side plan view, a partially cut away perspective view, and a top plan view of a toilet-bowl mounted sensor **300** for capturing and identifying an ingestible capsule. In some embodiments, the toilet-bowl mounted sensor **300** may form part of the system of Figure 1, for example as sensor **120.**

As seen in Figures 3A-3C, sensor **300** includes a receptacle **302** surrounded at a top end thereof by a sealing rim **304.** The sensor **300** is adapted to be placed within a toilet bowl **306,** at a portion thereof in which sealing rim **304** engages, and seals against, the entire circumference of the toilet bowl, such that any excrement expelled into the toilet bowl would be caught in receptacle **302.**

In some embodiments, the receptacle **302** is formed of a wire frame or a mesh frame, having gaps or openings large enough to allow water and excrement to pass through, but smaller than the diameter of the capsule to be caught in the receptacle, such that the capsule does not fall through the frame.

Extending along toilet bowl **306,** from the exterior thereof to a controller **308** of sensor **300** (described in further detail hereinbelow), is at least one conduit **310** for providing water flow to sensor **300,** and at least one wire **312** for providing electrical power to sensor **300** and/or for enabling communication of sensor **300** with a remote device, such as processor **110** of Figure 1.

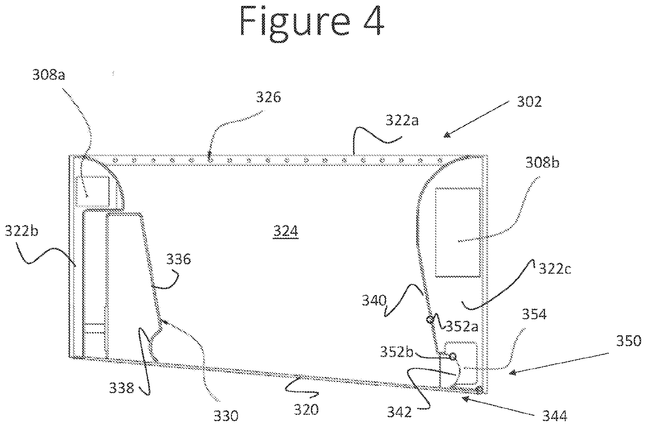

Reference is now made to Figure 4, which is a sectional view of an embodiment of receptacle **302** of toilet-bowl mounted sensor **300** in a stand-by mode.

As seen, receptacle **302** has a wire frame bottom surface **320** as described hereinabove, and side walls **322** surrounding a hollow **324.** At least one side wall, indicated in Figure 4 by reference numeral **322a** includes a plurality of water jets **326** which receive water from conduit **310** and are adapted to spray water into hollow **324,** in order to wash out excrement therefrom.

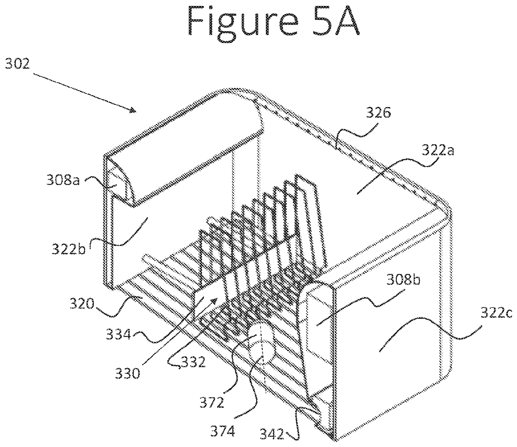

A pushing mechanism **330,** which in some embodiments includes a plurality of wire frame elements **332** (seen clearly in Figure 5A) connected to one another by a connector surface **334** (seen clearly in Figure 5A), is disposed within receptacle **302** and is movable relative to one of side walls **322b,** as explained in further detail hereinbelow. Pushing mechanism **330** is adapted to move back-and-forth along the receptacle **302** between side walls **322b** and **322c,** so as to provide mechanical force on excrement in the receptacle for breaking thereof into pieces that can be flushed out of the receptacle via the wire frame bottom **320.** In some embodiments, pushing mechanism **330** is further adapted to sense the Bristol stool measure of the excrement in receptacle **302,** for example by sensing the force exerted by the motor driving the pushing mechanism **330** for breaking down the excrement.

A side of pushing mechanism distal to the side wall **322b** includes a diagonal section **336** terminating in a generally hemispherical ingress **338,** which, together with a corresponding diagonal section **340** and generally hemispherical ingress **342** formed in an opposing side wall **322c** of receptacle **302** are adapted for capturing a vibrating ingestible capsule and for measurement and identification thereof, as described in further detail hereinbelow.

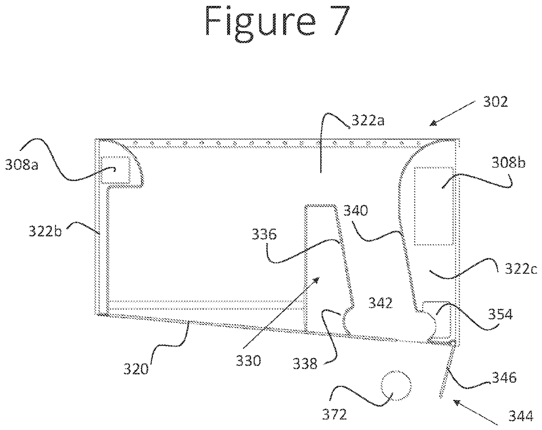

Disposed beneath ingress **342** is a capsule releasing mechanism **344,** adapted, following capturing of an expelled capsule and identification thereof, to release the capsule from receptacle **302** for flushing thereof down the toilet. In some embodiments, releasing mechanism **344** comprises a hatch **346** (seen clearly in Figure 7), hingedly connected to side wall **322c,** which is adapted to open and release a captured capsule following identification thereof, and to close back immediately following removal of the capsule.

It will be appreciated that though in the illustrated embodiment the bottom surface **320** is slanted toward side wall **322c** and ingress **342,** so as to allow gravity to assist a capsule in arriving at its capturing location as described in further detail hereinbelow, in some embodiments the bottom surface **320** may be horizontal and perpendicular to the side walls **322b** and **322c.**

Included within side wall **322c,** adjacent to ingress **342,** is a capsule identification mechanism **350,** which may include one or more sensors **352,** a vision system **354,** a communication system, and/or a weighing mechanism, for measuring the dimensions and/or weight of a captured capsule, and/or for uniquely identifying the capsule.

As described in further detail hereinbelow, sensors **352** may be contact sensors or distance sensors adapted to sense contact between, or a distance between, diagonal sections **336** and **340** and/or contact between a capsule captured between ingresses **338** and **342** and the ingress walls.

Vision system **354** has a field of view, and is typically adapted to view a wall of a captured capsule via the field of view, so as to uniquely identify the capsule. In some embodiments, the vision system may include a barcode reader adapted to identify a barcode printed onto the exterior of a captured capsule, a QR-code reader adapted to identify a QR-code printed onto the exterior of a captured capsule, an OCR mechanism adapted to scan and interpret text or an identification number printed onto the exterior of a captured capsule, or an image capturing and analysis mechanism adapted to capture an image of the exterior of the capsule and to analyze the captured image thereby to identify the capsule or a manufacturer thereof.

In some embodiments, the communication system includes an RFID tag reader adapted to read an RFID tag mounted on the exterior of a captured capsule, thereby to identify capsule. The communication system may be incorporated in the vision system or may be separate therefrom.

The weighing mechanism may be disposed on bottom surface **320** of the receptacle **302,** preferably adjacent to ingress **342,** so as to weigh a capsule captured between ingresses **338** and **342,** as described in further detail hereinbelow. In some embodiments, weighing mechanism is mounted onto releasing mechanism **344.**

Controller **308** of receptacle **302** may be disposed on or within one or more of side walls **322** and is adapted to control movement, and in some embodiments sensing capabilities, of pushing mechanism **330,** and to control operation of identification mechanism **350** and of releasing mechanism **344.** Additionally, controller **308** is adapted to provide power to pushing mechanism **330,** releasing mechanism **344** and identification mechanism **350.**

In some embodiments, controller **308** is adapted to receive input from weighing mechanism **344** and/or from identification mechanism **350,** so as to identify that a capsule has been captured, and the dimensions, weight, and/or identity of the captured capsule. In some embodiments, controller **308** includes a clock for identifying a time at which a capsule was captured and/or identified.

Typically, controller **308** is adapted to communicate with a remote location, such as processor **110,** via a communication module forming part of the controller and/or via a communication wire **310** extending from receptacle **302,** along toilet bowl **306** to the remote location. In some embodiments, the controller **308** may communicate the time at which the capsule was captured, and information regarding the capsule, such as its dimensions, weight, and/or identity, to the remote location, for use thereof. For example, in the embodiment of Figures 1 and 2, the information provided by the controller **308** may be used to identify a time that the capsule has spent in the GI tract of the subject, or an order at which a plurality of ingested capsules were expelled.

In some embodiments, the controller **308** may further communicate information relating to characteristics of the expelled fecal matter, such as a Bristol stool measure thereof, as sensed by pushing mechanism **330** or by the motor driving the pushing mechanism. In some such embodiments, the controller **308** only communicates the information relating to the fecal matter if a capsule was captured, or if a capsule of a specific type was captured.

In the embodiment illustrated in Figure 4, controller **308** has a first portion **308a** disposed within side wall **322b** and adapted to control and to provide power to pushing mechanism **330,** and a second portion **308b** disposed within side wall **322c** and adapted to control and to provide power to releasing mechanism **344** and identification mechanism **350.** However, any other arrangement of controller **308** is considered within the scope of the invention.

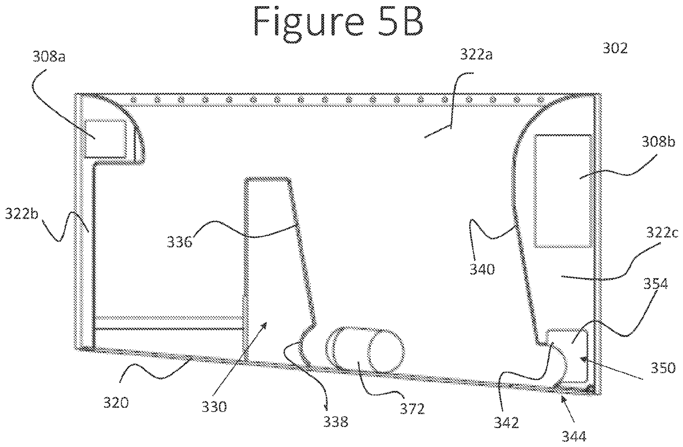

Reference is now made to Figures 5A and 5B, which are, respectively, a perspective view and a sectional view of the receptacle **302,** in an operational mode. As seen, pushing mechanism **330** is being pushed away from side wall **322b** and toward side wall **322c,** as indicated by arrow **370.** A vibrating ingestible capsule **372,** which was expelled from the body of a subject together with fecal matter thereof, is caught by receptacle **302,** and may be pushed by pushing mechanism **330** toward ingress **342** and identification mechanism **350.** In some embodiments, capsule **372** is generally ovoid, is symmetrical about a longitudinal axis **374,** and has a generally circular cross-section in a direction perpendicular to axis **374.**

It will be appreciated that pushing mechanism **330** may be pushed in the direction of arrow **370** and back in the opposite direction multiple times, so as to break down fecal matter caught in receptacle **302** for removal thereof via bottom surface **320.** In some embodiments, motion of pushing mechanism **330** is accompanied by water spray from jets **326** for washing away fecal matter and cleaning of the capsule, prior to pushing and/or capturing thereof.

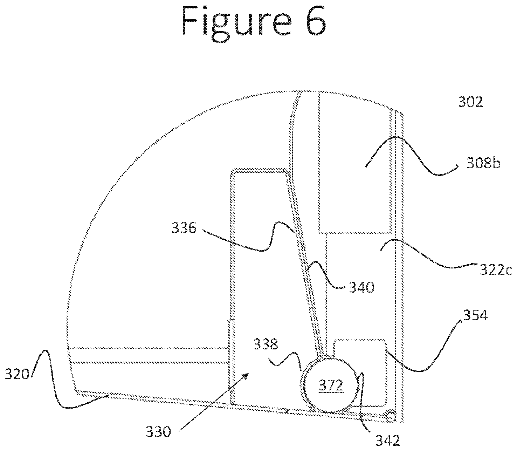

Reference is now made to Figure 6, which is a partial sectional view of receptacle **302** in a capsule capturing mode. As seen in Figure 6, pushing mechanism **330** is pushed distally from side wall **322b** until it engages side wall **322c,** such that diagonal sections **336** and **340** engage one another, and such that the capsule **372** is trapped between ingresses **338** and **342.** It is a particular feature of the present disclosure that the arrangement of the wire frame of bottom surface **320,** and ingress **338** of pushing mechanism **330,** cause capsule **372** to be captured such that longitudinal axis **374** is generally parallel to side wall **322c,** and such that the circular cross section of the capsule substantially fills the circular cross-section formed by ingresses **338** and **342.**

It is a particular feature of the teachings herein that capturing of the capsule, results in measurement of its dimensions. Specifically, sensors **352** of the identification mechanism **350** identify the dimensions of the capsule. Sensors **352a** mounted along section **340** identify whether or not diagonal sections **336** and **340** engage one another - if the diagonal sections do not engage one another when the capsule is captured, the capsule has a greater circumference than that of the circular cross section of the cavity between ingresses **338** and **342.** Sensors **352b** mounted along ingress **342** identify whether or not the circumference of the capsule engages the ingress wall - if the circumference of the capsule does not engage the ingress walls when the capsule is captured, the capsule has a smaller circumference than that of the circular cross section of the cavity between ingresses **338** and **342.** In some embodiments, weighing mechanism **358,** which may be disposed beneath ingresses **338** and **342,** identifies the weight of capsule **372** while the capsule is captured.

In some embodiments, the circular cross-section of the cavity between ingresses **338** and **342** may be specifically sized to match the cross section of a particular type of capsule, such that controller **308** may identify, based on input received from sensors **352,** whether or not the captured capsule is of the particular type. Additionally, even if the dimensions of captured capsule **372** match the expected dimensions, controller **308** may use the identified weight of the capsule to determine whether the capsule is indeed of the expected particular type. The controller **308** may report capture of the capsule, for example to processor **110,** if the capsule has the expected dimensions and weight, and may choose not to report capture of the capsule if it is differently sized or has a different weight.

When the capsule **372** is captured between ingresses **338** and **342,** vision system **354** may view a portion of the exterior of the capsule **372** via field of view **355,** and may thus uniquely identify the capsule. As described above, in one example, the capsule may have a barcode printed thereon, and the vision system includes a barcode reader adapted to identify the capsule by reading the barcode printed thereon. In another example, the capsule may have a QR-code printed thereon, and the visions system includes a QR-code reader adapted to identify the capsule by reading the code printed thereon. In yet another example, the capsule has a specific logo and/or an identification or serial number printed on the exterior thereof, and vision system **354** includes an OCR mechanism or other mechanism suitable for reading the logo and serial number, thereby to identify the capsule.

In some embodiments, identification of the capsule may alternately or additionally be accomplished by communication system **356.** For example, the communication system may include an RFID tag reader, which may identify an RFID tag mounted on the exterior of the captured capsule. Following identification of the capsule, the controller **308** may report capturing of the capsule, and information about the capsule, to a remote location, as described hereinabove. In some embodiments, the controller **308** may report the information only if the capsule is identified as belonging to a specific manufacturer, or as being of a specific type, for example as described hereinabove.

Reference is now made to Figure 7, which is a sectional view of receptacle **302** in a capsule releasing mode. As seen in Figure 7, following completion of the capsule identification, pushing mechanism **330** may be displaced away from side wall **322c** so as to release the capsule **372** from the hollow between ingresses **338** and **342,** and hatch **346** of releasing mechanism **344** may open, for example by rotating downwardly relative to bottom surface **320,** so as to allow the capsule **372** to drop out of receptacle **302b,** and to be flushed down the toilet. In some embodiments, the releasing mechanism is electrically controlled by controller **308,** such that hatch **346** recloses after a predetermined duration during which it is assumed that the capsule has been removed from the receptacle, or after sensors, for example on hatch **346,** identify releasing of the capsule.

Reference is now additionally made to Figure 8, which is a schematic flowchart of a method (which is not covered by the invention) for capturing and identifying an ingestible capsule that has been expelled into a toilet bowl, the method (which is not covered by the invention) utilizing the toilet-bowl mounted sensor **300** of any one of Figures 3A-7.

At an initial step **400,** excrement including an ingestible capsule is captured in a receptacle, such as receptacle **302,** of the sensor **300.** The pushing mechanism **330** is pushed forward (toward side wall **322c**) and back (away from side wall **322c** and toward side wall **322b),** so as to apply mechanical force to the excrement for breaking down thereof, as seen at step **402.**

Subsequently or simultaneously to the breaking action of the pushing mechanism, at step **404,** water jets **326** are activated to wash away excrement, which may be removed from the receptacle via the wire frame of bottom surface **320.**

In some embodiments, steps **402** and **404** may be repeated for a predetermined number of cycles, a predetermined duration, or until controller **308** determines that there is little or no residual excrement remaining on the capsule or in the receptacle.

At step **406,** following removal of excrement and cleaning of the capsule, the pushing mechanism **330** is displaced toward side wall **322c,** pushing the capsule in that direction, until the pushing mechanism **330** can move no further.

At step **408,** controller **308** assesses whether or not the pushing mechanism has engaged the side wall of the receptacle, and more specifically, whether or not diagonal sections **336** and **340** engage one another, for example using sensors **352a** (Figure 7).

If the pushing mechanism can move no further, but diagonal sections **336** and **340** do not engage one another, this is indicative of the captured capsule having a greater cross-section circumference than the expected capsule, and at step **410** the controller concludes that the captured capsule is not of the expected type. At step **412,** the releasing mechanism is operated to release the captured capsule, for example by opening hatch **346** as described hereinabove.

Otherwise, if at step **408** it is determined that the diagonal sections **336** and **340** engage one another, at step **414** controller **308** assesses whether the exterior surface of the captured capsule engages the surface of the cavity between ingresses **338** and **342,** for example by using sensor **352b** (Figure 7).

If the capsule does not engage the surface of the cavity when diagonal sections **336** and **340** engage one another, this is indicative of the captured capsule having a smaller cross-section circumference than the expected capsule, and the method (which is not covered by the invention) continues to step **410** where the controller concludes that the captured capsule is not of the expected type and to step **412** where the capsule is released from the receptacle.

If the capsule is determined to have the expected dimensions, controller **308** may receive from the weighing mechanism information relating to a weight of the captured capsule, and may determine, at step **418,** whether or not the measured weight of the capsule matches, or is within a predetermined margin of error, of an expected weight of the capsule. If the measured weight of the capsule does not match the expected weight, the method (which is not covered by the invention) continues to step **410** where the controller concludes that the captured capsule is not of the expected type and to step **412** where the capsule is released from the receptacle.

Otherwise, the controller concludes that the capsule is of the expected type, and at step **422** the identification mechanism uniquely identifies the capsule, for example by reading a barcode, QR code, unique identification number or string of characters, or an RFID tag located on the exterior surface of the capsule.

At step **424** the controller may report to a remote location, such as processor **110** of Figure 1, information relating to the capsule, such as the time the capsule was expelled from the body of the user or the time that the capsule was identified by the identification mechanism, and/or the unique identification of the capsule, which information may be used to better understand the subject's response to treatment with the capsule. In some embodiments, the controller may further report information regarding the fecal matter, such as a Bristol stool measure thereof, as identified by the pushing mechanism and as described hereinabove. As discussed in detail hereinabove, the reporting may be carried out by wired or wireless communication between the controller **308,** or another portion of the sensor **300,** and the remote location.

Subsequently or in parallel to such reporting, the capsule is released from the receptacle, at step **412.**

It will be appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A system (100) for treatment of a disorder in a gastrointestinal (GI) tract of a subject, the system comprising:
at least one input module (102) adapted to receive input from at least one of the subject, a medical practitioner treating the subject, and a care giver of the subject;
a computer readable memory adapted to store a subject profile (106) for the subject, said subject profile including subject data received as initial input by said at least one input module (102);
a database (108) including data relating to subjects treated for disorders in the gastrointestinal tract, to treatment protocols used for said subjects, and to the response to at least one specific subject to treatment of said at least one specific subject with a specific treatment protocol;
a first vibrating ingestible capsule (114a) adapted to be activated to implement a first vibration protocol defined in a first treatment protocol, and adapted, in an operative mode, to deliver vibrations to a wall of the GI tract of the subject in accordance with said first vibration protocol, thereby to treat the subject;
a second programmable vibrating ingestible capsule (114b) adapted to be programmed and activated to implement a second vibration protocol defined in a second treatment protocol, and adapted, in an operative mode, to deliver vibrations to a wall of the GI tract of the subject in accordance with said second vibration protocol, thereby to treat the subject;
a processor (110), functionally associated with said at least one input module (102), said computer readable memory, and said database (108); and
at least one sensor (120) adapted to provide to said processor (110) information regarding expelling of said first vibrating ingestible capsule (114a) from the body of the subject,
wherein said at least one input module (102) is adapted to receive feedback regarding a response of the subject to treatment in accordance with said first treatment protocol,
wherein said processor (110) is configured, based on at least one of said information regarding expelling and said feedback, to:
automatically obtain an updated recommendation for an updated recommended treatment protocol based on said data included in said database (108), said data relating to at least one other subject of said subjects; and
effect programming of said second programmable vibrating ingestible capsule (114b) to implement said second vibration protocol, said second treatment protocol including said second vibration protocol being based on said updated recommended treatment protocol.

2. The system of claim 1, wherein:
said first vibrating ingestible capsule (114a) comprises a first programmable vibrating ingestible capsule; and
said processor (110) is further configured to effect programming of said first programmable vibrating ingestible capsule (114a) to implement said first vibration protocol.

3. The system of claim 2, wherein said processor (110) is further configured, prior to effecting programming of said first programmable vibrating ingestible capsule (114a), to:
obtain an initial recommendation for an initial recommended treatment protocol based on at least one characteristic included in said subject data; and
generate said first treatment protocol based on said initial recommended treatment protocol.

4. The system of any one of claims 1 to 3, wherein said processor (110) is further configured to:
provide said updated recommended treatment protocol to a decision maker (118);
receive from said decision maker (118) an indication whether or not said second treatment protocol should be identical to said updated recommended treatment protocol; and
if said indication indicates that modifications are required to said updated recommended treatment protocol, modify said updated recommended treatment protocol in accordance with said required modifications to generate said second treatment protocol.

5. The system of claim 4, wherein said decision maker (118) is an electronic decision maker.

6. The system of claim 5, wherein said required modifications are within predetermined limits for electronically changing the initial recommended treatment protocol, the predetermined limits optionally being defined in the subject data.

7. The system of any one of claims 1 to 6, further comprising a capsule control unit (116) functionally associated with said processor and with said at least one second programmable vibrating ingestible capsule (114b), said capsule control unit (116) adapted to receive from said processor (110) said second vibration protocol and to program said second programmable vibrating ingestible capsule (114b) to implement said second vibration protocol.

8. The system of any one of claims 1 to 7, wherein said feedback comprises feedback received from the subject indicating times at which the subject experienced bowel movements during or following said treatment in accordance with said first treatment protocol.

9. The system of any one of claims 1 to 8, wherein said feedback comprises feedback received from the subject indicating a physical feeling experienced by the subject during or following said treatment in accordance with said first treatment protocol.

10. The system of any one of claims 1 to 9, wherein said feedback comprises feedback received from the subject indicating at least one characteristic of fecal matter excreted by the subject during or following said treatment in accordance with said first treatment protocol.

11. The system of any one of claims 1 to 10, wherein said information regarding expelling includes at least one of:
a time at which said first vibrating ingestible capsule (114a) was expelled from the body of the subject; and
an identification of said first vibrating ingestible capsule (114a).

12. The system of claim 11, wherein said at least one sensor comprises a toilet-bowl mounted sensor (300).

13. The system of any one of claims 1 to 12, wherein said processor (110) is further configured to use said subject data and said received feedback to update said database (108) to reflect said response of the subject to said treatment in accordance with said first treatment protocol.

## Patentansprüche

1. System (100) zur Behandlung einer Störung in einem Magen-Darm- (GI-)Trakt eines Subjekts, das System umfassend:
mindestens ein Eingabemodul (102), das angepasst ist, um Eingaben von mindestens einem von dem Subjekt, einem Arzt, der das Subjekt behandelt, und einer Pflegeperson des Subjekts zu empfangen;
einen computerlesbaren Speicher, der angepasst ist, um ein Subjektprofil (106) für das Subjekt zu speichern, wobei das Subjektprofil Subjektdaten beinhaltet, die als anfängliche Eingabe durch das mindestens eine Eingabemodul (102) empfangen werden;
eine Datenbank (108), die Daten beinhaltet, die sich auf Subjekte, die wegen Störungen in dem Magen-Darm-Trakt behandelt werden, auf Behandlungsprotokolle, die für diese Subjekte verwendet werden, und auf das Ansprechen mindestens eines spezifischen Subjekts auf eine Behandlung dieses mindestens einen spezifischen Subjekts mit einem spezifischen Behandlungsprotokoll beziehen;
eine erste vibrierende einnehmbare Kapsel (114a), die angepasst ist, um aktiviert zu werden, um ein erstes Vibrationsprotokoll zu implementieren, das in einem ersten Behandlungsprotokoll definiert ist, und angepasst ist, um in einem Betriebsmodus Vibrationen an eine Wand des GI-Trakts des Subjekts gemäß dem ersten Vibrationsprotokoll zuzuführen, um dadurch das Subjekt zu behandeln;
eine zweite programmierbare vibrierende einnehmbare Kapsel (114b), die so angepasst ist, dass sie programmiert und aktiviert wird, um ein zweites Vibrationsprotokoll zu implementieren, das in einem zweiten Behandlungsprotokoll definiert ist, und die so angepasst ist, dass sie in einem Betriebsmodus Vibrationen an eine Wand des GI-Trakts des Subjekt gemäß dem zweiten Vibrationsprotokoll zuführt, um dadurch den Subjekt zu behandeln;
einen Prozessor (110), der funktionell mit dem mindestens einen Eingabemodul (102), dem computerlesbaren Speicher und der Datenbank (108) assoziiert ist; und
mindestens einen Sensor (120), der angepasst ist, um dem Prozessor (110) Informationen über ein Ausstoßen der ersten vibrierenden einnehmbaren Kapsel (114a) aus dem Körper des Subjekts bereitzustellen,
wobei das mindestens eine Eingabemodul (102) angepasst ist, um eine Rückmeldung über ein Ansprechen des Subjekt auf eine Behandlung gemäß dem ersten Behandlungsprotokoll zu empfangen,
wobei der Prozessor (110) basierend auf mindestens einer der Informationen über das Ausstoßen und die Rückmeldung zu Folgendem konfiguriert ist:
automatisches Erlangen einer aktualisierten Empfehlung für ein aktualisiertes empfohlenes Behandlungsprotokoll basierend auf den in der Datenbank (108) beinhalteten Daten, wobei sich die Daten auf mindestens ein anderes Subjekt der Subjekte beziehen; und
Bewirken einer Programmierung der zweiten programmierbaren vibrierenden einnehmbaren Kapsel (114b), um das zweite Vibrationsprotokoll zu implementieren, wobei das zweite Behandlungsprotokoll das zweite Vibrationsprotokoll beinhaltet, das auf dem aktualisierten empfohlenen Behandlungsprotokoll basiert.

2. System nach Anspruch 1, wobei:
die erste vibrierende einnehmbare Kapsel (114a) eine erste programmierbare vibrierende einnehmbare Kapsel umfasst; und
der Prozessor (110) ferner konfiguriert ist, dass um ein Programmieren der ersten programmierbaren vibrierenden einnehmbaren Kapsel (114a) zu bewirken, um das erste Vibrationsprotokoll zu implementieren.

3. System nach Anspruch 2, wobei der Prozessor (110) vor Bewirken einer Programmierung der ersten programmierbaren vibrierenden einnehmbaren Kapsel (114a) ferner zu Folgendem konfiguriert ist:
Erlangen einer anfänglichen Empfehlung für ein anfängliches empfohlenes Behandlungsprotokoll basierend auf mindestens einem Merkmal, das in den Subjektdaten beinhaltet ist; und
Erzeugen des ersten Behandlungsprotokolls basierend auf dem anfänglichen empfohlenen Behandlungsprotokoll.

4. System nach einem der Ansprüche 1 bis 3, wobei der Prozessor (110) ferner zu Folgendem konfiguriert ist:
Bereitstellen des aktualisierten empfohlenen Behandlungsprotokolls an einen Entscheidungsträger (118);
Empfangen, von dem Entscheidungsträger (118) einer Angabe, ob das zweite Behandlungsprotokoll mit dem aktualisierten empfohlenen Behandlungsprotokoll identisch sein sollte; und
wenn die Angabe angibt, dass Modifikationen an dem aktualisierten empfohlenen Behandlungsprotokoll erforderlich sind, Modifizieren des aktualisierten empfohlenen Behandlungsprotokolls gemäß den erforderlichen Modifikationen, um das zweite Behandlungsprotokoll zu erzeugen.

5. System nach Anspruch 4, wobei der Entscheidungsträger (118) ein elektronischer Entscheidungsträger ist.

6. System nach Anspruch 5, wobei die erforderlichen Modifikationen innerhalb vorbestimmter Grenzen für ein elektronisches Ändern des anfänglichen empfohlenen Behandlungsprotokolls sind, wobei die vorbestimmten Grenzen optional in den Subjektdaten definiert sind.

7. System nach einem der Ansprüche 1 bis 6, ferner umfassend eine Kapselsteuereinheit (116), die funktionell mit dem Prozessor und mit der mindestens einen zweiten programmierbaren vibrierenden einnehmbaren Kapsel (114b) assoziiert ist, wobei die Kapselsteuereinheit (116) angepasst ist, um das zweite Vibrationsprotokoll von dem Prozessor (110) zu empfangen und die zweite programmierbare vibrierende einnehmbare Kapsel (114b) zu programmieren, um das zweite Vibrationsprotokoll zu implementieren.

8. System nach einem der Ansprüche 1 bis 7, wobei die Rückmeldung eine von dem Subjekt empfangene Rückmeldung umfasst, die Zeiten angibt, an denen das Subjekt während oder nach der Behandlung gemäß dem ersten Behandlungsprotokoll Stuhlgang hatte.

9. System nach einem der Ansprüche 1 bis 8, wobei die Rückmeldung eine von dem Subjekt empfangene Rückmeldung umfasst, die ein von dem Subjekt während oder nach der Behandlung gemäß dem ersten Behandlungsprotokoll empfundenes körperliches Gefühl angibt.

10. System nach einem der Ansprüche 1 bis 9, wobei die Rückmeldung eine von dem Subjekt empfangene Rückmeldung umfasst, die mindestens ein Merkmal der von dem Subjekt während oder nach der Behandlung gemäß dem ersten Behandlungsprotokoll ausgeschiedenen Fäkalien angibt.

11. System nach einem der Ansprüche 1 bis 10, wobei die Informationen über ein Ausstoßen mindestens eines von Folgenden beinhalten:
einen Zeitpunkt, an dem die erste vibrierende einnehmbare Kapsel (114a) aus dem Körper des Subjekts ausgestoßen wurde; und
eine Identifizierung der ersten vibrierenden einnehmbaren Kapsel (114a).

12. System nach Anspruch 11, wobei der mindestens eine Sensor einen an der Toilettenschüssel montierten Sensor (300) umfasst.

13. System nach einem der Ansprüche 1 bis 12, wobei der Prozessor (110) ferner konfiguriert ist, um die Subjektdaten und die empfangene Rückmeldung zu verwenden, um die Datenbank (108) zu aktualisieren, um das Ansprechen des Subjekts auf die Behandlung gemäß dem ersten Behandlungsprotokoll widerzuspiegeln.

## Revendications

1. Système (100) permettant le traitement d'un trouble d'un tractus gastro-intestinal (GI) d'un sujet, le système comprenant :
au moins un module d'entrée (102) adapté à la réception d'une entrée provenant d'au moins un parmi le sujet, un médecin traitant le sujet et/ou un soignant du sujet ;
une mémoire lisible par ordinateur adaptée au stockage d'un profil de sujet (106) pour le sujet, ledit profil de sujet comprenant des données de sujet reçues en tant qu'entrée initiale par ledit au moins un module d'entrée (102) ;
une base de données (108) comprenant des données relatives à des sujets traités pour des troubles dans le tractus gastro-intestinal, aux protocoles de traitement utilisés pour lesdits sujets, et à la réponse d'au moins un sujet spécifique au traitement dudit au moins un sujet spécifique avec un protocole de traitement spécifique ;
une première capsule ingérable vibrante (114a) adaptée à l'activation pour mettre en oeuvre un premier protocole de vibration défini dans un premier protocole de traitement, et adaptée, dans un mode de fonctionnement, à la délivrance de vibrations à une paroi du tractus GI du sujet conformément audit premier protocole de vibration, pour ainsi traiter le sujet ;
une seconde capsule ingérable vibrante programmable (114b) adaptée à la programmation et à l'activation de manière à mettre en oeuvre un second protocole de vibration défini dans un second protocole de traitement, et adaptée, dans un mode de fonctionnement, à la délivrance de vibrations à une paroi du tractus GI du sujet conformément audit second protocole de vibration, pour ainsi traiter le sujet ;
un processeur (110), fonctionnellement associé audit au moins un module d'entrée (102), à ladite mémoire lisible par ordinateur et à ladite base de données (108) ; et
au moins un capteur (120) adapté à la fourniture audit processeur (110) d'informations concernant l'expulsion de ladite première capsule ingérable vibrante (114a) du corps du sujet,
ledit au moins un module d'entrée (102) étant adapté à la réception d'un retour d'information concernant une réponse du sujet au traitement conformément audit premier protocole de traitement,
ledit processeur (110) étant configuré, sur la base d'au moins un paramètre parmi lesdites informations concernant l'expulsion et ledit retour d'information, pour :
obtenir automatiquement une recommandation mise à jour pour un protocole de traitement recommandé mis à jour sur la base desdites données comprises dans ladite base de données (108), lesdites données concernant au moins un autre sujet parmi lesdits sujets ; et
effectuer une programmation de ladite seconde capsule ingérable vibrante programmable (114b) de manière à mettre en oeuvre ledit second protocole de vibration, ledit second protocole de traitement comprenant ledit second protocole de vibration étant basé sur ledit protocole de traitement recommandé mis à jour.

2. Système selon la revendication 1 :
ladite première capsule ingérable vibrante (114a) comprenant une première capsule ingérable vibrante programmable ; et
ledit processeur (110) étant en outre configuré pour effectuer la programmation de ladite première capsule ingérable vibrante programmable (114a) de manière à mettre en oeuvre ledit premier protocole de vibration.

3. Système selon la revendication 2, ledit processeur (110) étant en outre configuré, avant d'effectuer la programmation de ladite première capsule ingérable vibrante programmable (114a), pour :
obtenir une recommandation initiale pour un protocole de traitement recommandé initial sur la base d'au moins une caractéristique comprise dans lesdites données du sujet ; et
générer ledit premier protocole de traitement sur la base dudit protocole de traitement recommandé initial.

4. Système selon l'une quelconque des revendications 1 à 3, ledit processeur (110) étant en outre configuré pour :
fournir ledit protocole de traitement recommandé mis à jour à un décideur (118) ;
recevoir de la part dudit décideur (118) une indication stipulant si ledit second protocole de traitement doit ou non être identique audit protocole de traitement recommandé mis à jour ; et
si ladite indication indique que des modifications sont nécessaires audit protocole de traitement recommandé mis à jour, modifier ledit protocole de traitement recommandé mis à jour conformément auxdites modifications requises pour générer ledit second protocole de traitement.

5. Système selon la revendication 4, ledit décideur (118) étant un décideur électronique.

6. Système selon la revendication 5, lesdites modifications requises se trouvant dans des limites prédéfinies pour modifier électroniquement le protocole de traitement recommandé initial, les limites prédéfinies étant éventuellement définies dans les données du sujet.

7. Système selon l'une quelconque des revendications 1 à 6, comprenant en outre une unité de commande de capsule (116) fonctionnellement associée audit processeur et à ladite au moins une seconde capsule ingérable vibrante programmable (114b), ladite unité de commande de capsule (116) étant adaptée à la réception en provenance dudit processeur (110) dudit second protocole de vibration et à la programmation de ladite seconde capsule ingérable vibrante programmable (114b) pour mettre en oeuvre ledit second protocole de vibration.

8. Système selon l'une quelconque des revendications 1 à 7, ledit retour d'information comprenant un retour d'information reçu de la part du sujet indiquant les moments auxquels le sujet a expérimenté des mouvements intestinaux pendant ou après ledit traitement conformément audit premier protocole de traitement.

9. Système selon l'une quelconque des revendications 1 à 8, ledit retour d'information comprenant un retour d'information reçu de la part du sujet indiquant une sensation physique éprouvée par le sujet pendant ou après ledit traitement conformément audit premier protocole de traitement.

10. Système selon l'une quelconque des revendications 1 à 9, ledit retour d'information comprenant le retour d'information reçu de la part du sujet indiquant au moins une caractéristique des matières fécales excrétées par le sujet pendant ou après ledit traitement conformément audit premier protocole de traitement.

11. Système selon l'une quelconque des revendications 1 à 10, lesdites informations concernant l'expulsion comprenant au moins l'un des éléments suivants :
le moment auquel ladite première capsule ingérable vibrante (114a) a été expulsée du corps du sujet ; et
l'identification de ladite première capsule ingérable vibrante (114a).

12. Système selon la revendication 11, ledit au moins un capteur comprenant un capteur monté sur cuvette de toilettes (300).

13. Système selon l'une quelconque des revendications 1 à 12, ledit processeur (110) étant en outre configuré pour utiliser lesdites données du sujet et ledit retour d'information reçu de manière à mettre à jour ladite base de données (108) afin de refléter ladite réponse du sujet audit traitement conformément audit premier protocole de traitement.
